# NEUE EUROPÄISCHE PATENTSCHRIFT

(11) **EP 0 209 071 B2**
(45) Veröffentlichungstag und Bekanntmachung des Hinweises auf die Entscheidung über den Einspruch: **15.07.1998**
(45) Hinweis auf die Patenterteilung: 15.11.1989
(21) Anmeldenummer: 86109361.5
(22) Anmeldetag: 09.07.1986
(51) Int. Cl.: A61L 2/18, G02C 13/00, A01N 59/00

(54) **Kontaktlinsenpflegesatz**
Cleaning set for contact lenses
Kit pour l'entretien des lentilles de contact

(30) Priorität: 10.07.1985 DE 3524659; 11.09.1985 DE 3532433
(43) Veröffentlichungstag der Anmeldung: 21.01.1987
(73) Patentinhaber: Novartis AG, 4058 Basel (CH)
(72) Erfinder: Schäfer, Horst, Dr. rer. nat., D-8750 Aschaffenburg-Obernau (DE); Ludwig, Gerhard, Dr., D-6534 Stromberg (DE); Sunderdiek, Rainer, Dr., D-8750 Aschaffenburg-Schweinheim (DE)
(74) Vertreter: Diehl, Hermann O. Th., Dr.

(56) Entgegenhaltungen:
- EP-A- 0 124 461
- EP-A- 0 139 994
- EP-A- 0 147 100
- US-A- 3 912 451

## Beschreibung

Pflegesätze für weiche und harte Kontaktlinsen dienen zur Reinigung, Desinfizierung und Stabilisierung der optischen Eigenschaften und Verbesserung des Tragekomforts der Kontaktlinsen. Für die Desinfektion und Reinigung von Kontaktlinsen hat das Wasserstoffperoxid als antimikrobielle Substanz in neuerer Zelt an Bedeutung gewonnen. Wasserstoffperoxid besitzt gegenüber Chlorhexidinsalzen oder Quecksilber-organischen Verbindungen, z. B. Thiomersal, welche ebenfalls zur Desinfektion von Kontaktlinsen verwendet werden, eine bessere antimikrobiologische Wirkung insbesondere bezüglich der relativ resistenten Aspergillusarten. Die bekannten, bei der Reinigung von Kontaktlinsen verwendeten Wasserstoffperoxidlösungen sind 3 %-ige Peroxidlösungen (DE-PS-2 425 714). Hieraus resultiert, daß nach der Behandlung der Kontaktlinsen mit der Wasserstoffperoxidlösung relativ große Restmengen an der Linse haften, die nach dem Einsetzen zu mehr oder weniger starken Schleimhautreizungen führen. Es ist daher erforderlich, nach der Behandlung mit einer Wasserstoffperoxidlösung die Linse mit einer Neutralisationslösung zu behandeln, durch die das Wasserstoffperoxid in Wasser und Sauerstoff aufgespalten wird. Aus der vorstehend genannten DE-PS-2 425 714 ist es bekannt, mit Hilfe von insbesondere Schwermetallkatalysatoren nach der Durchführung der Sterilisierbehandlung der Linse Restwasserstoffperoxidmengen durch Zersetzung zu beseitigen. Es ist hierbei die Anwendung zweier verschiedener Behandlungsschritte erforderlich, nämlich zunächst der Behandlungsschritt der Sterilisierung der Linse und nach dem Wegschütten der Sterilisierlösung der Behandlungsschritt der Beseitigung des Restwasserstoffperoxids in einem den Katalysator enthaltenden wäßrigen System. Die Handhabung der Linse bei der Pflege und Reinigung gestaltet sich daher relativ umständlich. Schwierigkeiten bereitet auch die Wahl geeigneter Katalysatoren, welche bei der Zersetzung der Restwasserstoffperoxidmengen zum Einsatz kommen.

Aus der EP-A-82 798 sind als Katalysatoren zur Zersetzung des zur Desinfektion und Reinigung von Kontaktlinsen eingesetzten Wasserstoffperoxids nach der Durchführung der Reinigungs- und Desinfektionsbehandlung der Einsatz von enzymatischen Peroxidasen, insbesondere Katalase, als Zersetzungskatalysatoren bekannt. Hierbei wird ebenfalls zunächst die Wasserstoffperoxidlösung nach der Desinfektionsbehandlung abgegossen und durch eine neutrale Lösung ersetzt, die die neutralen Peroxidasen enthält. Auch hierbei müssen zwei verschiedene Behandlungsschritte zur Anwendung gebracht werden, wodurch die Pflegebehandlung der Linse umständlich wird. Die gleichzeitige Anwendung des Wasserstoffperoxids mit dem Zersetzungskatalysator verbietet sich, weil dann das Wasserstoffperoxid, bevor es ausreichend sterilisierend auf die Linse zur Einwirkung kommen kann, durch die Anwesenheit des Zersetzungskatalysators in Sauerstoff und Wasser zersetzt wird, so daß eine ausreichende Sterilisierbehandlung zur Abtötung der Keime, welche eine bis zu vierstündige Einwirkung des Wasserstoffperoxids auf die Linse, insbesondere auf die weiche Linse, erfordert, nicht gewährleistet wird.

Gemäss der Lehre der WO 86/07264 ist ein Verfahren zur Behandlung von Kontaktlinsen mit Wasserstoffperoxid bekannt, bei dem die Behandlung in Gegenwart eines immobilisierten Enzyms durchgeführt wird, welches die Zersetzung von Wasserstoffperoxid katalysiert. Es findet sich jedoch kein Hinweis auf ein Retardierungsmittel.

In der EP-A-250 427 wird eine Zusammensetzung beschrieben, die sowohl eine Wasserstoffperoxid abgebende Verbindung als Desinfektionsmittel enthält, als auch eine weitere Verbindung, die das Desinfektionsmittel inaktiviert. Die inaktivierende Verbindung liegt in einer Form vor, aus der sie verzögert abgageben wird. Beide genannten Komponenten liegen, zusammen mit einem Farbindikator, stets in fester Form kombiniert vor.

Die EP-A-139,994 beschreibt einen Kontaktlinsenpflegesatz, der aus den folgenden Komponenten besteht (siehe Anspruch 14):
a) einem sterilisierend wirkenden Wasserstoffperoxid;
b) einer beschichteten Tablette, die eine oder mehr Wasserstoffperoxid zersetzende ("neutralizing") Verbindungen und ein oder mehrere wasserlösliche Mittel enthält, das u.a. einen pH-Wert von 6.5 bis 8.5 ergibt.
Die Beschichtung löst sich in der Wasserstoffperoxid-Lösung auf und gibt die zersetzende(n) Verbindung(en) und das (die) wasserlösliche(n) Mittel erst nach Ablauf des Desinfektionszeitraumes frei.

Aufgabe der Erfindung ist es, einen verbesserten Kontaktlinsenpflegesatz der gattungsgemäßen Art zu schaffen, der eine einstufige Behandlung der Linse bei der Sterilisierung bzw. Reinigung ermöglicht, wobei eine ausreichende Einwirkungszeit des Wasserstoffperoxids auf die Linse und die erforderliche Beseitigung von Restwasserstoffperoxidmengen an der Linse erreicht werden.

Diese Aufgabe wird erfindungsgemäß gelöst durch die im Kennzeichen der Ansprüche 1, 2 oder 3 angegebenen Merkmale.

Die Unteransprüche kennzeichnen Weiterbildungen der Erfindung.

Durch die Erfindung wird die Pflege der Kontaktlinse - es kann sich hierbei um harte oder weiche Kontaktlinsen handeln - erheblich erleichtert, weil nur noch eine einstufige Behandlung erforderlich ist. Hierbei werden die Linse und gleichzeitig das katalysatorhaltige Neutralisationsmittel, welches mit einem die zeitverzögerte Freigabe des Neutralisationsmittels und/oder Katalysators steuernden Retardierungsmittel kombiniert ist, in die sterilisierend wirkende Wasserstoffperoxidlösung eingelegt. Das Retardierungsmittel, welches mit der Wasserstoffperoxidlösung beim Eintauchen in Berührung kommt, gibt nach einer bestimmten, z. B. durch die Dicke einer Umhüllung einstellbaren Einwirkungszeit (insbesondere bis zu vier Stunden) der Wasserstoffperoxidlösung auf die zu behandelnde Kontaktlinse das katalysatorhaltige Neutralisationsmittel frei. Durch das frei gegebene katalysatorhaltige Neutralisationsmittel erfolgt dann automatisch ohne weiteres Zutun des die Kontaktlinse pflegenden Kontaktlinsenträgers die Einwirkung des Neutralisationsmittels auf die in ihrem stabilen Zustand saure (pH < 4) Wasserstoffperoxidlösung und die Zersetzung dieser sterilisierend wirkenden Wasserstoffperoxidlösung durch den Katalysator in dem Behandlungsbehälter, in weichem gleichzeitig die zu behandelnde Linse, das sterilisierende Wasserstoffperoxid und das Neutralisationsmittel eingebracht wurden.

Beispiele für die Zusammensetzung der Wasserstoffperoxidlösung und des katalysator- bzw. enzymhaltigen Neutralisationsmittels sind in der DE-OS-3 410 400 angegeben.

Eine weitere geeignete Zusammensetzung für das Neutralisationsmittel ist folgende:
3 - 12 mg geeignete Puffersubstanz(en) oder -mischung z. B. Alkaliphosphate, -borate, -citrate, Glycin
40 - 70 mg Neutralelektrolyt (z. B. NaCl, KCl)
5 - 10 mg Alkalihydrogencarbonat
5 - 10 mg wasserlösliches Polymer z. B. Polyvinylpyrrolidon
0,2 - 1 mg Katalysator (Katalase)
pro Einzeldosis.

Diese Menge reicht aus, um 7 ml einer Wasserstoffperoxidlösung zu zersetzen und zu neutralisieren und auf eine Osmolarität von 270 - 320 mOsmol einzustellen. 7 ml entsprechen dem Volumen eines Standard-Kontaktlinsenetuis bzw. Kontaktlinsenbehandlungskörpers.

Ein Beispiel für eine weitere spezielle Zusammensetzung des Neutralisationsmittels ist folgendes:
5,6 mg Kaliumdihydrogenphosphat
8 mg di-Natriumhydrogenphosphat
52,4 mg NaCl
7 mg Natriumhydrogencarbonat
4,8 mg Polyvinylpyrrolidon K25
0,3 mg Katalysator

Die vorstehend angegebenen Zusammensetzungen für das Neutralisationsmittel können als Kapselfüllungen verwendet werden, wobei die Kapsel als das Retardierungsmittel ausgebildet ist, welches das katalysatorhaltige Neutralisationsmittel umgibt. Die Kapsel besteht aus einem wasserlöslichen Polymer, insbesondere Polyvinylalkohol.

Das Neutralisationsmittel kann auch zu einer Tablette geformt sein, die mit einer wasserlöslichen Umhüllung zur zeitlich verzögerten Auflösung der Tablette versehen ist. Die wasserlösliche Umhüllung kann aus einem in saurem Milieu löslichen Polymer z. B. einem Polymerisat aus Dimethylaminomethacrylat und neutralen Methacrylsäureestern bestehen. Die Umhüllung kann auch aus einem pH-neutrallöslichen Polymer bestehen. Hierfür geeignete Polymere sind beispielsweise lösliche Celluloseether z. B. Methylcellulose, Methylhydroxypropylcellulose, Methylhydroxyethylcellulose, Hydroxypropylcellulose, Hydroxyethylcellulose, Na-Carboxymethylcellulosen; Celluloseacetatphthalat; Hydroxypropylmethylcellulosephthalat; Polymerisat aus Methacrylsäure und Methacrylsäureestern;

Überzug aus einer wäßrigen Dispersion eines gemischten Polymerisats von Methacrylsäure und Methacrylsäureestern; Überzug aus einer wäßrigen Dispersion von Celluloseacetatphthalat; Mischpolymerisat aus Methylvinylether und Maleinsäureanhydrid und Polyvinylalkohole.

Diesen Polymeren können zur Steuerung der zeitlichen Verzögerung geeignete Polyalkohole zugesetzt werden, insbesondere in einer Menge von 0,2 - 1 mg/Tablette, wobei als Polyalkohole 1,2-Propylenglykol, Polyethylenglykole und Citronensäureester geeignet sind.

Der Überzug aus dem wasserlöslichen Polymer kann mit bekannten Verfahren z. B. durch Sprühbefilmung in Dragierkesseln, fluidized-bed Verfahren (Wirbelschicht, Wurster Verfahren) oder in geschlossenen Systemen hergestellt werden. Die Überzugsmenge beträgt insbesondere 2,0 - 5,0 mg/Tablette.

Ein Beispiel für eine Tablettenzusammensetzung, welche das katalysatorhaltige Neutralisationsmittel bildet, ist folgendes:
11,2 mg di-Natriumhydrogenphosphat
55,8 mg NaCl
4,8 mg Polyvinylpyrrolidon K25
0,2 mg Katalysator (Katalase)

Ein Beispiel für ein sauerlösliches Polymer als Umhüllung ist folgendes:
1,9 mg Methacrylsäureester
0,4 mg Hydroxypropylmethylcellulose
0,2 mg Polyethylenglykol (1000)

Ein Ausführungsbeispiel für einen Überzugsfilm aus neutrallöslichem Polymer ist folgendes:
2,2 mg Hydroxypropylmethylcellulosephthalat
0,3 mg Polyethylenglykol (1000)

Es ist auch möglich, das zu einer Tablette geformte katalysatorhaltige Neutralisationsmittel in einer wasserlöslichen Folie z. B. aus Polyvinylalkohol, welche als Retardierungsmittel wirkt, einzuschweißen. Der Polyvinylalkoholfolie sind Weichmacheranteile von mehrwertigen Alkoholen und Wasser mit einem Anteil von 3,5 % bis 5 % zugesetzt, wodurch eine zeitlich verzögerte Auflösung der Folie bewirkt wird. Die Dicken der Folien können von 30 - 150 µm reichen. Die Gasdurchlässigkeit (DIN 53 380) beträgt 0,9 - 1,4 cm³ · m⁻² · d⁻¹ · bar-¹.

Die Folien sind im Temperaturbereich von 140°C - 190°C mit einem Anpreßdruck von 4 - 5 bar verschweißbar.

Das als Tablette geformte katalysatorhaltige Neutralisationsmittel kann auch mit einer unlöslichen jedoch semipermeablen Membran überzogen sein. Diese Membran kann ebenfalls durch bekannte Überzugsverfahren beispielsweise Sprühbefilmung, Wirbelschichtverfahren oder in geschlossenen Systemen aufgebracht werden und zwar in einer Menge von 3 bis 10 mg/Tablette. Die semipermeable Membran kann beispielsweise wie folgt gebildet werden aus einer organischen Lösung von Ethylcellulose, einer wäßrigen Ethylcellulosedispersion, einem Copolymerisat aus Acryl/Methacrylsäureester mit Trimethylammoniummethacrylat, einer wäßrigen Dispersion von gemischten Methacrylsäuremethylestern und Methacrylsäureethylestern, wobei zur Steuerung der Diffusionsrate geeignete Weichmacher in einer Menge von insbesondere 1,0 - 5,0 mg/Tablette zugesetzt werden können. Als Weichmacher eignen sich 1,2-Propylenglykol, Polyethylenglykole verschiedenen Molgewichts und Citronensäureester. Ein Zusammensetzungsbeispiel für die semipermeable Membran ist wie folgt:
3,8 mg Ethylcellulose N22
1,2 mg Polyethylenglykol (6000)

Auf dem medizinischen Sektor ist es bei der Verabreichung von Arzneistoffen bekannt, therapeutische Systeme in Form von Tabletten, semipermeablen Membranen und Matrizen zu verwenden, die in ein bestimmtes Zielgebiet eines Biosystems gebracht werden, um eine über einen längeren Zeitraum hin konstante Arzneistoffabgabe zu erreichen. Im Gegensatz dazu wird bei der Erfindung erreicht, daß der das Wasserstoffperoxid zersetzende Katalysator zusammen mit dem sterilisierend wirkenden Wasserstoffperoxid bei der Kontaktlinsenbehandlung gleichzeitig zur Anwendung gebracht werden kann, ohne daß die für einen bestimmten Behandlungszeitraum, beispielsweise von vier Stunden erwünschte Sterilisierwirkung des Wasserstoffperoxids durch den Wasserstoffperoxid zersetzenden Katalysator beeinträchtigt wird. Die zersetzende Wirkung des Katalysators auf das Wasserstoffperoxid tritt erst nach Ablauf der gewünschten Behandlungszeit auf.

Die Behandlungslösung, bei welcher die Freigabe des katalysatorhaltigen Neutralisationsmittels erfolgt, kann einen pH-Wert von etwa 7 bis 7,5 insbesondere 7,3 und eine Osmolarität von ca. 300 mosmol besitzen.

Durch Farbumschlag, insbesondere durch Anwendung eines Farbindikators (pH/redox-Indikator), bei 7,0 bis 7,5, insbesondere 7,3, wird dem Benutzer angezeigt, daß das für die Sterilisierbehandlung verwendete Wasserstoffperoxid zersetzt ist. Hierfür eignen sich insbesondere hochmolekulare Farbstoffe, die nicht in das Linsenmaterial eindringen.

Damit der Katalysator erst nach Beendigung der Desinfektions- bzw. Sterilisierbehandlung der Linse seine Wirkung ausübt, kann dieser Katalysatorzusatz zusammen mit im Neutralisationsmittel vorhandenen Hilfsstoffen, die zur Neutralisierung der Wasserstoffperoxidlösung dienen, in einer Umhüllung verpackt sein, durch die gewährleistet wird, daß erst nach der erforderlichen Behandlungszeit (bis zu vier Stunden) der Katalysator zur Zersetzung des Wasserstoffperoxids freigesetzt wird. Hierzu empfehlen sich eine Reihe von Möglichkeiten.

Die Umhüllung bzw. Verpackung des Katalysators und der Hilfsstoffe besitzt die Form einer Kapsel, die bei dem pH-Wert, bei welchem die Wasserstoffperoxidlösung stabil ist, d. h. bei einem pH-Wert von 4 bis etwa 5, wasserlöslich ist, jedoch zu keiner Ausflockung oder Ausfällung bei neutraler Lösung, d. h. bei etwa pH 7,3, führt.

Von Vorteil ist es außerdem, wenn die Kapsel mit einer oder zwei oder gegebenenfalls auch mehr Laserperforationen versehen ist, durch die beim Kontakt mit der Wasserstoffperoxidlösung die Hilfsstoffe freigegeben werden, wobei nach Erreichen eines pH-Wertes von etwa 7 die Kapsel sich völlig auflöst, um dann den Katalysator freizusetzen.

Der Katalysator kann auch als Enzymprill vorliegen, der zusammen mit den Hilfsstoffen in die Kapsel gefüllt ist, die die vorstehend genannten Auflösungseigenschaften hat.

Das katalysatorhaltige Neutralisationsmittel kann auch als mit einem Überzug versehene Tablette ausgebildet sein, bei der bei einer verzögerten Auflösung des Überzugs zunächst die Hilfsstoffe zur Veränderung des pH-Werts der Wasserstoffperoxidlösung und dann bei etwa einem pH-Wert von 7 der Katalysator freigesetzt werden.

Ferner eignet sich für das katalysatorhaltige Neutralisationsmittel eine Zweischichttablette, bei der die eine Schicht aus löslichen Salzen zur Neutralisation der Wasserstoffperoxidlösung dient und die andere Schicht den Katalysator enthält, wobei die Hilfsstoffe zuerst freigesetzt werden können.

Ferner kann das Neutralisationsmittel eine semipermeable Membran als Umhüllung aufweisen, durch die Wasser zur Auflösung der Hilfsstoffe in das Innere eindringen und aus welcher die gelösten Salze durch den osmotischen Druck nach außen gelangen. Die semipermeable Membran kann hierzu eine Perforation aufweisen oder sie kann durch den osmotischen Druck zerstört werden, wobei dann die Hilfsstoffe und der Katalysator freigesetzt werden. Der Katalysator kann hierbei als umhülltes Enzymprodukt vorliegen.

Ferner eignet sich ein Zweischichtsystem mit löslicher und semipermeabler Membran für die zeitlich versetzte Freigabe der Hilfsstoffe und des Katalysators. Ein nicht lösliches Membransystem könnte bei der erneuten Anwendung ausgetauscht werden.

Ein geeignetes Zweischichtsystem kann so aufgebaut sein, daß der Katalysator von der semipermeablen Membran umhüllt ist, an deren Außenseite die Hifsstoffe liegen. Eine beim pH-Wert (< 4) der stabilen Wasserstoffperoxidlösung wasserlösliche Umhüllung umhüllt dabei die Hilfsstoffe und die den Katalysator einschließende semipermeable Membran.

Die Zersetzung des Wasserstoffperoxids kann durch einen Farbindikator nachweisbar sein. Es kann sich hierbei um die Zugabe eines pH/Redox-Indikators handeln, der in die weichen hydrophilen Kontaktlinsen nicht eintritt und der physiologisch unbedenklich ist. Der Farbindikator kann so ausgestaltet sein, daß er bei pH 7 in Anwesenheit von H₂O₂ gefärbt ist oder bei pH 7 in Abwesenheit von H₂O₂ farblos ist.

Der Behälter, in welchem die Kontaktlinsenpflege bzw. -behandlung durchgeführt wird, kann eine Entlüftungsmöglichkeit beispielsweise in Form eines Bunsenventils oder dgl. besitzen, durch die der freigesetzte Sauerstoff bei der Neutralisierung und bei der Zersetzung des Wasserstoffperoxids entweichen kann.

Die beiliegenden Figuren dienen zur weiteren Erläuterung der Erfindung. Es zeigen:
Fig. 1 bis 5 verschiedene Ausführungsbeispiele für Wasserstoffperoxid und Zersetzungskatalysator enthaltende Systeme;
Fig. 6 den zeitlichen Ablauf der Behandlung einer Kontaktlinse mit einem der Ausführungsbeispiele des Kontaktlinsenpflegesatzes;

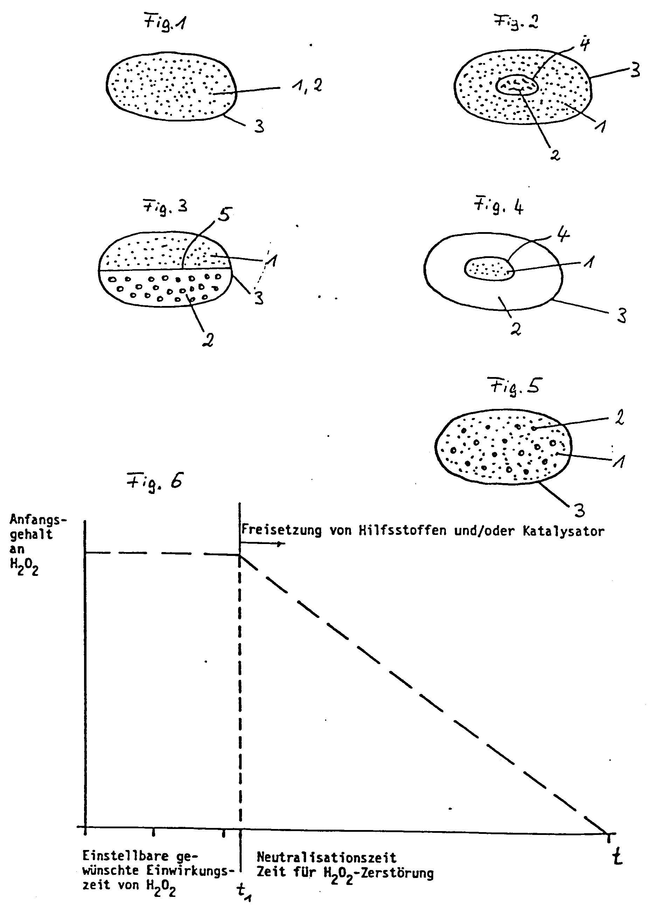

Bei dem in der Fig. 1 dargestellten Ausführungsbeispiel sind Hilfsstoffe 1 und ein wasserstoffperoxidspaltender Katalysator 2, in Form von Katalase, statistisch verteilt in einer äußeren Hülle 3 vorgesehen. Die äußere Hülle 3 kann nach einer bestimmten Einwirkungszeit des Wasserstoffperoxids, das bei der Kontaktlinsenpflege sterilisierend auf die zu behandelnde Kontaktlinse wirkte wasserlöslich oder semipermeabel sein. Durch die Dicke der Hülle läßt sich die Einwirkungszeit (bis zu vier Stunden) des Wasserstoffperoxids auf die Linse einstellen, bevor dann durch die Auflösung der äußeren Hülle 3 oder durch die Semipermeabilität dieser Hülle soviel Katalase und Hilfsstoffe freigesetzt sind, daß die sterilisierend wirkende Wasserstoffperoxidlösung neutralisiert und durch die katalytische Zersetzung beseitigt wird. Die Freisetzung der Hilfsstoffe und des Katalysators kann bei diesem Ausführungsbeispiel gleichzeitig erfolgen.

Bei dem in der Fig. 2 dargestellten Ausführungsbeispiel werden zuerst die Hilfsstoffe 1 freigesetzt, wodurch die sterilisierend wirkende Wasserstoffperoxidlösung neutralisiert wird und danach kommt der das Wasserstoffperoxid zerstörende Katalysator 2, in Form von Katalase, zur Anwendung. Hierzu ist die äußere Hülle 3 vorgesehen, die nach einer bestimmten Einwirkungszeit des Wasserstoffperoxids löslich ist oder welche entsprechend semipermeabel ausgebildet ist, und die die Hilfsstoffe 1 umgibt. Die Hilfsstoffe 1 umgeben den Katalysator 2, welcher seinerseits von einer inneren Hülle 4 umgeben ist, welche ebenfalls löslich oder semipermeabel ausgebildet sein kann.

Bei dem in der Fig. 3 dargestellten Ausführungsbeispiel liegen der Katalysator 2 und die Hilfsstoffe 1 nebeneinander und sind von einer gemeinsamen äußeren Hülle 3, welche wasserlöslich oder semipermeabel ist, umgeben. Die Hilfsstoffe 1 und der Katalysator 2 können dabei von einer Trennwand 5 voneinander getrennt sein.

Bei der in der Fig. 4 dargestellten Ausführungsform erfolgt zuerst die Freisetzung des Katalysators 2, der in Form einer viskosen Lösung vorliegen kann, und danach die Freisetzung der Hilfsstoffe 1. Der Katalysator 2 wird dabei von der äußeren Hülle 3 umgeben und die Hilfsstoffe 1 von der inneren Hülle 4. Die Hilfsstoffe 1 liegen bei dieser Ausführungsform innen und werden von dem Katalysator 2 umgeben.

Bei der in der Fig. 5 dargestellten Ausführungsform sind der Katalysator 2 als Katalysatorprill und die Hilfsstoffe 1 statistisch verteilt in der äußeren Hülle 3, welche löslich oder semipermeabel sein kann, vorhanden.

In der Fig. 6 ist graphisch der zeitliche Ablauf der Behandlung der Kontaktlinse mit dem Wasserstoffperoxid und die anschließende Zerstörung des Wasserstoffperoxids nach der gewünschten Einwirkungszeit t₁ dargestellt. Die Einwirkungszeit des Wasserstoffperoxids kann beispielsweise durch die Dicke der Umhüllung oder auch durch entsprechende Materialwahl für die Umhüllung festgelegt werden. Auf der Ordinate ist der Gehalt an Wasserstoffperoxid aufgetragen, wobei der Anfangsgehalt des Wasserstoffperoxids bis zur Beendigung der Einwirkungszelt t₁ im wesentlichen konstant bleibt. Nach Freisetzen der Hilfsstoffe und/oder des Katalysators nimmt der Gehalt an Wasserstoffperoxid im Behandlungsbehälter rasch ab.

## Patentansprüche

1. Kontaktlinsenpflegesatz mit zwei Behandlungsmitteln, von denen das eine Behandlungsmittel sterilisierend wirkendes Wasserstoffperoxid und das andere Behandlungsmittel ein katalysatorhaltiges Neutralisationsmittel zur Neutralisation und Zersetzung des Wasserstoffperoxides nach der Sterilisierbehandlung einer Kontaktlinse ist, dadurch gekennzeichnet, daß es sich bei dem Katalysator um Katalase handelt, und daß das Neutralisationsmittel und/oder die Wasserstoffperoxid zersetzende Katalase von einer Umhüllung umschlossen ist, die in Kontakt mit der Wasserstoffperoxidlösung nach einer bestimmten Einwirkungszeit der Wasserstoffperoxidlösung aus die zu behandelnde Kontaktlinse das katalasehaltige Neutralisationsmittel freigibt, wobei die Umhüllung aus einem Polymer besteht, das in neutralem Milieu löslich ist, wobei das Polymer ausgewählt ist aus der Gruppe Hydroxypropylmethylcellulosephthalat, ein Polymerisat aus Methacrylsäure und Methacrylsäureestern und einem Mischpolymerisat aus Methylvinylether und Maleinsäureanhydrid und Polyvinylalkohole.

2. Kontaktlinsenpflegesatz mit zwei Behandlungsmitteln, von denen das eine Behandlungsmittel sterilisierend wirkendes Wasserstoffperoxid und das andere Behandlungsmittel ein katalysatorhaltiges Neutralisationsmittel zur Neutralisation und Zersetzung des Wasserstoffperoxides nach der Sterilisierbehandlung einer Kontaktlinse ist, dadurch gekennzeichnet, daß es sich bei dem Katalysator um Katalase handelt, und daß das Neutralisationsmittel und/oder die Wasserstoffperoxid zersetzende Katalase mit einem die zeitverzögerte Freigabe steuernden Retardierungsmittel kombiniert sind, wobei das Retardierungsmittel die Form einer wasserlöslichen Umhüllung für das katalysatorhaltige Neutralisationsmittel hat und aus einem in saurem oder neutralem Milieu wasserlöslichen Polymer besteht und dem wasserlöslichen Polymer zur Steuerung der Retardierungszeit Polyalkohole zugesetzt sind.

3. Kontaktlinsenpflegesatz mit zwei Behandlungsmitteln, von denen das eine Behandlungmittel sterilisierend wirkendes Wasserstoffperoxid und das andere Behandlungsmittel ein katalysatorhaltiges Neutralisationsmittel zur Neutralisation und Zersetzung des Wasserstoffperoxides nach der Sterilisierbehandlung einer Kontaktlinse ist, dadurch gekennzeichnet, daß es sich bei dem Katalysator um Katalase handelt, und daß das Neutralisationsmittel und/oder die Wasserstoffperoxid zersetzende Katalase mit einem die zeitverzögerte Freigabe steuernden Retardierungsmittel kombiniert sind, wobei die Katalase nach der Neutralisation der Wasserstoffperoxidlösung freigesetzt ist.

4. Kontaktlinsenpflegesatz nach einem der Ansprüche 1 bis 3, dadurch gekennzeichnet, daß das Neutralisationsmittel neben der Katalase Hilfsstoffe aufweist, die die saure Wasserstoffperoxidlösung neutralisieren.

5. Kontaktlinsenpflegesatz nach Anspruch 4, dadurch gekennzeichnet, daß die Hilfsstoffe NaH₂PO₄, Na₂HPO₄ und NaC1 Puffersubstanzen, Filmbildner und benetzende Stoffe sind.

6. Kontaktlinsenpflegesatz nach einem der Ansprüche 1 bis 3, dadurch gekennzeichnet, daß die Zersetzung des Wasserstoffperoxids durch einen Farbindikator nachweisbar ist.

7. Kontaktlinsenpflegesatz nach Anspruch 2, dadurch gekennzeichnet, daß die Polyalkohole 1,2-Propylenglykol, Polyethlenglykol, Citronensäureester sind.

8. Kontaktlinsenpflegesatz nach Anspruch 2, dadurch gekennzeichnet, daß das in saurem Milieu lösliche Polymer ein Polymerisat aus Dimethylaminomethacrylat und neutralen Methacrylsäureestern ist.

9. Kontaktlinsenpflegesatz nach Anspruch 2, dadurch gekennezeichnet, daß das in neutralem Milieu lösliche Polymer eine Verbindung aus der Gruppe lösliche Celluloseether, Celluloseacetatphthalat, Hydroxypropylmethylcellulosephthalat, ein Polymerisat aus Methacrylsäure und Methacrylsäureestern, ein Mischpolymerisat aus Methylvinylether und Maleinsäureanhydrid und Polyvinylalkohole ist.

10. Kontaktlinsenpflegesatz nach Anspruch 9, dadurch gekennzeichnet, daß die löslichen Celluloseether Methylcellulose, Methylhydroxypropylcellulose, Methylhydroxyethylcellulose, Hydroxypropylcellulose, Hydroxyethylcellulose und Na*-*Carboxymethylcellulose sind.

11. Kontaktlinsenpflegesatz nach Anspruch 2, dadurch gekennzeichnet, daß die Umhüllung in Form einer wasserlöslichen Polyvinylalkoholfolie vorliegt und Weichmacheranteile von mehrwertigen Alkoholen und Wasser von 3,5 % bis 5 % enthält.

12. Kontaktlinsenpflegesatz nach Anspruch 2, dadurch gekennzeichnet, daß das Retardierungsmittel in Form einer semipermeablen, das katalysatorhaltige Neutralisationsmittel umhüllenden Membran vorliegt, die aus Ethylcellulose oder einem Copolymerisat aus Acryl/Methacrylsäureester mit Trimethylammoniummethacrylat oder einer Mischung von Methacrylsäuremethylestern und Methacrylsäureethylestern besteht, wobei zur Steuerung der Diffusionsrate der Membran die Weichmacher 1,2-Propylenglycol oder Polyethylenglycole oder Citronensäureester sind.

13. Kontaktlinsenpflegesatz nach Anspruch 3, dadurch gekennzeichnet, daß das Retardierungsmittel die Form einer wasserlöslichen Umhüllung hat, welche bei dem pH-Wert (< 4), bei welchem die Wasserstoffperoxidlösung stabil ist, wasserlöslich oder semipermeabel ist.

14. Kontaktlinsenpflegesatz nach Anspruch 3, dadurch gekennzeichnet, daß das katalasehaltige Neutralisationsmittel als mit einem Überzug versehene Tablette ausgebildet ist, derart, daß bei einer verzögerten Auflösung des Überzugs zunächst die Hilfsstoffe zur Veränderung des pH-Wertes der Wasserstoffperoxidlösung und dann bei etwa einem pH-Wert von 7 die Katalase freigesetzt sind.

15. Kontaktlinsenpflegesatz nach Anspruch 14, dadurch gekennzeichnet, daß das Neutralisationsmittel als Zweischichttablette ausgebildet ist, bei der die eine Schicht aus löslichen Salzen zur Neutralisation der Wasserstoffperoxidlösung dient und die andere Schicht der Katalysator ist.

16. Kontaktlinsenpflegesatz nach Anspruch 3, dadurch gekennzeichnet, daß das Neutralisationsmittel von einer semipermeablen Membran umhüllt ist, durch die Wasser zur Auflösung der Hilfsstoffe in das Innere eindringen und aus welcher die gelösten Salze durch den osmotischen Druck nach außen gelangen.

17. Kontaktlinsenpflegesatz nach Anspruch 16, dadurch gekennzeichnet, daß die semipermeable Membran eine oder mehrere Perforationen aufweist.

18. Kontaktlinsenpflegesatz nach Anspruch 17, dadurch gekennzeichnet, daß die semipermeable Membran durch den osmotischen Druck zerstörbar ist.

19. Kontaktlinsenpflegesatz nach Anspruch 13 und einem der Ansprüche 16 bis 18, dadurch gekennzeichnet, daß der Katalysator von der semipermeablen Membran umhüllt ist, an deren Außenseite die Hilfsstoffe liegen und daß die beim pH-Wert (< 4) der stabilen Wasserstoffperoxidlösung wasserlösliche bzw. semipermeable Umhüllung die Hilfsstoffe und die den Katalysator einschließende semipermeable Membran umhüllt.

## Claims

1. Contact lens care kit with two treatment agents, one of which is hydrogen peroxide with sterilising action and the other is a catalyst-containing neutralising agent which neutralises and breaks down the hydrogen peroxide after the sterilising treatment of a contact lens, characterised in that the catalyst in question is catalase and the neutralising agent and/or the catalase which breaks down the hydrogen peroxide is enclosed by a coating which releases the catalase-containing neutralising agent when in contact with the hydrogen peroxide solution after the hydrogen peroxide solution has acted on the contact lens to be treated for a certain period, whereby the coating consists of a polymer which is soluble in a neutral medium, whereby the polymer is selected from the group hydroxypropylmethyl cellulose phthalate, a polymerisate of methacrylic acid and methacrylic acid esters and a mixed polymerisate of methyl vinyl ether and maleic acid anhydride and polyvinyl alcohols.

2. Contact lens care kit with two treatment agents, one of which is hydrogen peroxide with sterilising action and the other is a catalyst-containing neutralising agent which neutralises and breaks down the hydrogen peroxide after the sterilising treatment of a contact lens, characterised in that the catalyst in question is catalase and the neutralising agent and/or the catalase which breaks down the hydrogen peroxide are combined with a retarding agent which controls the time-delayed release, whereby the retarding agent has the form of a water-soluble coating for the catalyst-containing neutralising agent and consists of a polymer that is water-soluble in an acidic or neutral medium, and polyalcohols are added to the water-soluble polymer in order to control the retarding period.

3. Contact lens care kit with two treatment agents, one of which is hydrogen peroxide with sterilising action and the other is a catalyst-containing neutralising agent which neutralises and breaks down the hydrogen peroxide after the sterilising treatment of a contact lens, characterised in that the catalyst in question is catalase and the neutralising agent and/or the catalase which breaks down the hydrogen peroxide are combined with a retarding agent which controls the time-delayed release, whereby the catalase is released after neutralisation of the hydrogen peroxide solution.

4. Contact lens care kit according to one of claims 1 to 3, characterised in that, apart from the catalase, the neutralising agent has excipients which neutralise the acidic hydrogen peroxide solution.

5. Contact lens care kit according to claim 4, characterised in that the excipients are NaH₂PO₄, Na₂HPO₄ and NaCl buffer substances, film-forming components and wetting substances.

6. Contact lens care kit according to one of claims 1 to 3, characterised in that breakdown of the hydrogen peroxide can be detected by means of a colour indicator.

7. Contact lens care kit according to claim 2, characterised in that the polyalcohols are 1,2-propylene glycol, polyethylene glycol, citric acid esters.

8. Contact lens care kit according to claim 2, characterised in that the polymer which is soluble in an acidic medium is a polymerisate of dimethylamino methacrylate and neutral methacrylic acid esters.

9. Contact lens care kit according to claim 2, characterised in that the polymer which is soluble in a neutral medium is a compound from the group soluble cellulose ethers, cellulose acetate phthalate, hydroxypropylmethyl cellulose phthalate, a polymerisate of methacrylic acid and methacrylic acid esters, a mixed polymerisate of methyl vinyl ether and maleic acid anhydride and polyvinyl alcohols.

10. Contact lens care kit according to claim 9, characterised in that the soluble cellulose ethers are methyl cellulose, methyl hydroxypropyl cellulose, methyl hydroxyethyl cellulose, hydroxypropyl cellulose, hydroxyethyl cellulose and Na carboxymethyl cellulose.

11. Contact lens care kit according to claim 2, characterised in that the coating exists in the form of a water-soluble polyvinyl alcohol film and contains softener constituents of polyvalent alcohols and water in portions of 3.5% to 5%.

12. Contact lens care kit according to claim 2, characterised in that the retarding agent exists in the form of a semi-permeable membrane coating the catalyst-containing neutralising agent, the membrane consisting of ethyl cellulose or a copolymerisate of acrylic/methacrylic acid ester with trimethylammonium methacrylate or a mixture of methacrylic acid methyl esters and methacrylic acid ethyl esters, whereby in order to control the diffusion rate of the membrane, the softeners are 1,2-propylene glycol or polyethylene glycols or citric acid esters.

13. Contact lens care kit according to claim 3, characterised in that the retarding agent has the form of a water-soluble coating which is water-soluble or semi-permeable at the pH value (< 4) at which the hydrogen peroxide solution is stable.

14. Contact lens care kit according to claim 3, characterised in that the catalase-containing neutralising agent is designed as a coated tablet, so that when there is delayed disintegration of the coating, first of all the excipients which modify the pH value of the hydrogen peroxide solution are released, and then at a pH value of 7 the catalase is released.

15. Contact lens care kit according to claim 14, characterised in that the neutralising agent is designed as a two-layer tablet, in which one layer consisting of soluble salts serves to neutralise the hydrogen peroxide solution and the other layer is the catalyst.

16. Contact lens care kit according to claim 3, characterised in that the neutralising agent is enclosed by a semi-permeable membrane through which water penetrates to the interior in order to dissolve the excipients, and from which the dissolved salts reach the exterior by means of osmotic pressure.

17. Contact lens care kit according to claim 16, characterised in that the semi-permeable membrane has one or more perforations.

18. Contact lens care kit according to claim 17, characterised in that the semi-permeable membrane can be broken down by osmotic pressure.

19. Contact lens care kit according to claim 13 and one of claims 16 to 18, characterised in that the catalyst is enclosed by the semi-permeable membrane, on the exterior of which are the excipients, and that the coating which is water-soluble or semi-permeable at the pH value (< 4) of the stable hydrogen peroxide solution encloses the excipients and the semi-permeable membrane enclosing the catalyst.

## Revendications

1. Nécessaire d'entretien de lentilles de contact avec deux agents de traitement, dont l'un est un peroxyde d'hydrogène à action stérilisante et l'autre est un agent neutralisant contenant un catalyseur pour la neutralisation et la décomposition du peroxyde d'hydrogène après le traitement de stérilisation d'une lentille de contact, caractérisé en ce que le catalyseur est une catalase et en ce que l'agent neutralisant et/ou la catalase décomposant le peroxyde d'hydrogène est entouré d'un enrobage qui, au contact de la solution de peroxyde d'hydrogène après un temps d'action déterminé de la solution de peroxyde d'hydrogène sur la lentille de contact à traiter, libère l'agent neutralisant contenant la catalase, l'enrobage étant constitué d'un polymère qui est soluble en milieu neutre, le polymère étant choisi parmi le phtalate d'hydroxypropylméthylcellulose, un polymère de l'acide méthacrylique et d'esters de l'acide méthacrylique et un polymère mixte de méthylvinyléther et d'un anhydride de l'acide maléique et d'alcools polyvinyliques.

2. Nécessaire d'entretien de lentilles de contact avec deux agents de traitement, dont l'un est un peroxyde d'hydrogène à action stérilisante et l'autre est un agent neutralisant contenant un catalyseur pour la neutralisation et la décomposition du peroxyde d'hydrogène après le traitement de stérilisation d'une lentille de contact, caractérisé en ce que le catalyseur est une catalase et en ce que l'agent neutralisant et/ou la catalase décomposant le peroxyde d'hydrogène sont combinés avec un agent retardant réglant la libération retardée dans le temps, l'agent retardant ayant la forme d'un enrobage soluble dans l'eau pour l'agent neutralisant contenant le catalyseur et étant constitué d'un polymère soluble dans l'eau en milieu acide ou neutre et des polyalcools étant ajoutés au polymère soluble dans l'eau pour régler le temps de retard.

3. Nécessaire d'entretien de lentilles de contact avec deux agents de traitement, dont l'un est un peroxyde d'hydrogène à action stérilisante et l'autre est un agent neutralisant contenant un catalyseur pour la neutralisation et la décomposition du peroxyde d'hydrogène après le traitement de stérilisation d'une lentille de contact, caractérisé en ce que le catalyseur est une catalase et en ce que l'agent neutralisant et/ou la catalase décomposant le peroxyde d'hydrogène sont combinés avec un agent retardant réglant la libération retardée dans le temps, la catalase étant libérée après la neutralisation de la solution de peroxyde d'hydrogène.

4. Nécessaire d'entretien de lentilles de contact selon l'une des revendications 1 à 3, caractérisé en ce que l'agent neutralisant, outre la catalase, présente des additifs qui neutralisent la solution acide de peroxyde d'hydrogène.

5. Nécessaire d'entretien de lentilles de contact selon la revendication 4, caractérisé en ce que les additifs sont NaH₂PO₄, Na₂HPO₄ et NaCl, des substances tampons, des substances formant des films et des agents mouillants.

6. Nécessaire d'entretien de lentilles de contact selon l'une des revendications 1 à 3, caractérisé en ce que la décomposition du peroxyde d'hydrogène est mise en évidence par un indicateur coloré.

7. Nécessaire d'entretien de lentilles de contact selon la revendication 2, caractérisé en ce que les polyalcools sont le 1,2-propylèneglycol, le polyéthylèneglycol, les esters de l'acide citrique.

8. Nécessaire d'entretien de lentilles de contact selon la revendication 2, caractérisé en ce que le polymère soluble en milieu acide est un polymère de diméthylaminométhacrylate et d'esters neutres de l'acide méthacrylique.

9. Nécessaire d'entretien de lentilles de contact selon la revendication 2, caractérisé en ce que le polymère soluble en milieu neutre est un composé du groupe des éthers de cellulose solubles, des acétato-phtalates de cellulose, des phtalates d'hydroxypropylméthylcellulose, d'un polymère de l'acide méthacrylique et des esters de l'acide méthacrylique, d'un polymère mixte de méthylvinyléther et d'un anhydride de l'acide maléique et d'alcools polyvinyliques.

10. Nécessaire d'entretien de lentilles de contact selon la revendication 9, caractérisé en ce que les éthers de cellulose solubles sont la méthylcellulose, la méthylhydroxypropylcellulose, la méthylhydroxyéthylcellulose, l'hydroxypropylcellulose, l'hydroxyéthylcellulose et la carboxyméthylcellulose sodique.

11. Nécessaire d'entretien de lentilles de contact selon la revendication 2, caractérisé en ce que l'enrobage se présente sous la forme d'une feuille d'alcool polyvinylique soluble dans l'eau et contient des fractions plastifiantes d'alcools polyvalents et d'eau de 3,5% à 5%.

12. Nécessaire d'entretien de lentilles de contact selon la revendication 2, caractérisé en ce que l'agent retardant se présente sous la forme d'une membrane semi-perméable enrobant l'agent neutralisant contenant le catalyseur, qui se compose d'éthylcellulose ou d'un copolymère d'esters de l'acide acrylique/méthacrylique avec du méthacrylate de triméthylammonium ou d'un mélange d'esters méthyliques de l'acide méthacrylique et d'esters éthyliques de l'acide méthacrylique, pour régler la vitesse de diffusion de la membrane, les plastifiants étant le 1,2-propylèneglycol ou le polyéthylèneglycol ou des esters de l'acide citrique.

13. Nécessaire d'entretien de lentilles de contact selon la revendication 3, caractérisé en ce que l'agent retardant se présente sous la forme d'un enrobage soluble dans l'eau qui est soluble dans l'eau ou semi-perméable à un pH (< 4) auquel la solution de peroxyde d'hydrogène est stable.

14. Nécessaire d'entretien de lentilles de contact selon la revendication 3, caractérisé en ce que l'agent neutralisant contenant la catalase est formé en comprimés munis d'un enrobage, afin que lors d'une dissolution retardée de l'enrobage, les additifs servant à modifier le pH de la solution de peroxyde d'hydrogène sont d'abord libérés, puis, la catalase, à un pH d'environ 7.

15. Nécessaire d'entretien de lentilles de contact selon la revendication 14, caractérisé en ce que l'agent neutralisant se présente sous la forme de comprimés à deux couches, une couche étant un sel soluble pour la neutralisation de la solution de peroxyde d'hydrogène et l'autre couche étant le catalyseur.

16. Nécessaire d'entretien de lentilles de contact selon la revendication 3, caractérisé en ce que l'agent neutralisant est enrobé par une membrane semi-perméable à travers laquelle l'eau pénètre à l'intérieur pour la dissolution des additifs et hors de laquelle les sels dissous parviennent à l'extérieur sous l'action de la pression osmotique.

17. Nécessaire d'entretien de lentilles de contact selon la revendication 16, caractérisé en ce que la membrane semi-perméable présente une ou plusieurs perforations.

18. Nécessaire d'entretien de lentilles de contact selon la revendication 17, caractérisé en ce que la membrane semi-perméable est destructible par pression osmotique.

19. Nécessaire d'entretien de lentilles de contact selon la revendication 13 et l'une des revendications 16 à 18, caractérisé en ce que le catalyseur est enrobé par la membrane semi-perméable, à l'extérieur de laquelle se trouvent les additifs et en ce que, à un pH (< 4) de la solution stable de peroxyde d'hydrogène, l'enrobage soluble dans l'eau ou semi-perméable entoure les additifs et la membrane semi-perméable contenant le catalyseur.
